# EUROPEAN PATENT SPECIFICATION

(11) **EP 1 379 305 B1**
(45) Date of publication and mention of the grant of the patent: **12.04.2006**
(21) Application number: 02719816.7
(22) Date of filing: 13.02.2002
(51) Int. Cl.: A61M 15/00

(54) **MEDICAMENT DISPENSER**
MEDIKAMENTENSPENDER
DISTRIBUTEUR DE MEDICAMENT

(30) Priority: 20.04.2001 GB 0109717
(43) Date of publication of application: 14.01.2004
(73) Proprietor: GLAXO GROUP LIMITED, Greenford, Middlesex UB6 ONN (GB)
(72) Inventor: DAVIES, Michael, Birsha, Ware, Herts SG12 0DP (GB)
(74) Representative: Rice, Jason Neale
(86) International application number: PCT/EP2002/001483
(87) International publication number: WO 2002/085436

(56) References cited:
- EP-A- 0 075 548
- EP-A- 0 341 967
- WO-A-99/25405

## Description

The present invention relates to a medicament dispenser. In particular, the present invention relates to a nasal inhalator having a protective cover.

Drugs for treating respiratory and nasal disorders are frequently administered in aerosol formulations through the mouth or nose. One widely used method for dispensing such aerosol drug formulations involves formulating the drug as a suspension or a solution in a liquefied gas propellant. The suspension/solution is stored in a sealed canister capable of withstanding the pressure required to maintain the propellant as a liquid. The suspension/solution is dispersed by activation of a dose-metering valve affixed to the canister.

A metering valve generally comprises a metering chamber which is of a set volume and is designed to administer per actuation an accurate predetermined dose of medicament As the suspension is forced from the canister through the dose metering valve by the high vapour pressure of the propellant, the propellant rapidly vaporizes leaving a fast moving cloud of very fine particles of the drug formulation. This cloud of particles is directed into the nose or mouth of the patient by a channelling device such as a cylinder or open-ended cone. Concurrently with the activation of the aerosol dose-metering valve, the patient inhales the drug particles into the lungs or nasal cavity. Systems of dispensing drugs in this way are known as "metered dose inhalers" (MDI's). See Peter Byron, Respiratory Drug Delivery, CRC Press, Boca Raton, FL (1990) for a general background on this form of therapy.

Patients often rely on medication delivered by MDI's for rapid treatment of respiratory disorders that are debilitating and in some cases even life threatening. Therefore, it is essential that the prescribed dose of aerosol medication delivered to the patient consistently meet the specifications claimed by the manufacturer and comply with the requirements of the FDA and other regulatory authorities. That is, every dose delivered from the canister must be the same within dose tolerances.

Conventional metering valves for use with pressurized containers comprise a valve stem coaxially slidable within a valve member defining an annular metering chamber, and outer and inner seals operative between the respective outer and inner ends of the valve stern and the valve member to seal the metering chamber therebetween. The valve stem is hollow whereby in a non-dispensing position of the valve stem, the metering chamber is connected to the container and charged with product therefrom. The valve stem is movable to a dispensing position wherein the metering chamber is isolated from the container and vented to the atmosphere for the discharge of product. At rest, the valve can be biased towards the non-dispensing or dispensing position by, for example, a spring, or by the internal pressure exerted by the propellant composition.

In order to substantially alleviate or prevent contamination of the channelling component that delivers the medicament to the nose or mouth, many inhalers have a protective cover.

Such protective covers may take the form of an axially displaceable lid, or a lid connected to the body of the inhaler device via a flexible connecting member.

EP-A-075548 discloses an aerosol dispensing device with a protective cover which takes the form of a casing suspended to rotate about the body of the device.

WO-A-99/25405 discloses a medicament dispenser according to the pre-characterising part of claim 1 hereof.

However, such devices can be confusing to use, and moreover, the casing can interfere with proper use of the dispensing device. Commonly, latching devices and interacting locking members have to be fitted to the casing and or the body of the device in order to prevent accidental closing of the casing during dispensing of the medicament

An object of the present invention is to provide a medicament dispenser having a cover which is urged to remain in the dispensing position during dispensing of the medicament. It is another object of the present invention to provide a device having grips for the user that serve at least three functions; firstly, the grips indicate the mode of operation of the device; secondly, the grips direct the user how to use the device; thirdly, the grips afford the user better handling of the device which ultimately ensures correct dispensing of the medicament and proper dosing.

Accordingly, in one aspect, the invention provides a medicament dispenser comprising:-
(i) a medicament container having a dosing valve associated therewith such that medicament is dispensed in response to a force applied to an actuation point on said medicament container to depress said container relative to said valve;
(ii) a body for housing said container, said body having a discharge nozzle for discharging the medicament therethrough; and
(iii) a protective cover in which said body rotates between a storage position whereby said nozzle is covered and a dispensing position whereby said nozzle is exposed;
wherein said cover comprises a grip positioned such that as the medicament is dispensed, opposing forces applied to said actuation point and to said grip do not generate a turning moment urging said body to return to said storage position.

As used herein the term *actuation point* typically refers to a position on the top of the medicament container and lies outside of the body housing the medicament container.

The invention thus provides a dispenser whereby the application of opposing forces to the top of the medicament container and to the grip on the cover, urges the body to remain in the dispensing position without the need for complex latching mechanisms between the body and cover. This differs from inhalers presently available where the cover is held in the user's hand at a position towards the rear of the inhaler body and force applied to the top of the medicament container urges the body to rotate within the cover towards the storage position.

Preferably, the medicament container is an aerosol container.

Typically, the medicament dispenser takes the form of a nasal inhalator.

Preferably, the body further comprises a lip member to restrain movement of the body with respect to the cover.

Typically, the lip member restrains further onward rotation of the body in the cover after the body has rotated from the storage position to the dispensing position.

Thus, the lip member may take the form of a protrusion on the lowermost part of the body beneath the discharge nozzle. Thus, when the body is rotated with respect to the cover to the dispensing position, the lip prevents continued rotation in the same direction.

In one embodiment, the body further comprises a grip.

Preferably, the grip is positioned at the distal side of the body which is opposite the discharge nozzle. Thus, this grip member may indicate to the user where to apply force to the body in order to rotate the body from the storage to the dispensing position.

The grip on the cover and/or the body may comprise ridges, ribs, depressions or mounds.

Suitably, the grip on the cover and/or the body may be made from plastics, and/or fabric, and/or rubber.

Typically, the cover is suspended on the body on an axis transverse to a longitudinal axis through the medicament container.

Preferably, the axis is substantially central to the body and to the cover.

The cover and the body may further comprise interacting members to retain the body in a dispensing or storage position.

In another aspect, the invention provides a protective cover for use with a medicament dispenser as defined *supra.*

The invention will now be described further with reference to the accompanying drawings, in which:-
Figure 1 shows an exploded view of a body and a cover in accordance with the invention;
Figure 2 shows a schematic view of a medicament dispenser in accordance with the present invention in the storage position; and,
Figure 3 shows a schematic view of the medicament dispenser of figure 1 in the dispensing position.

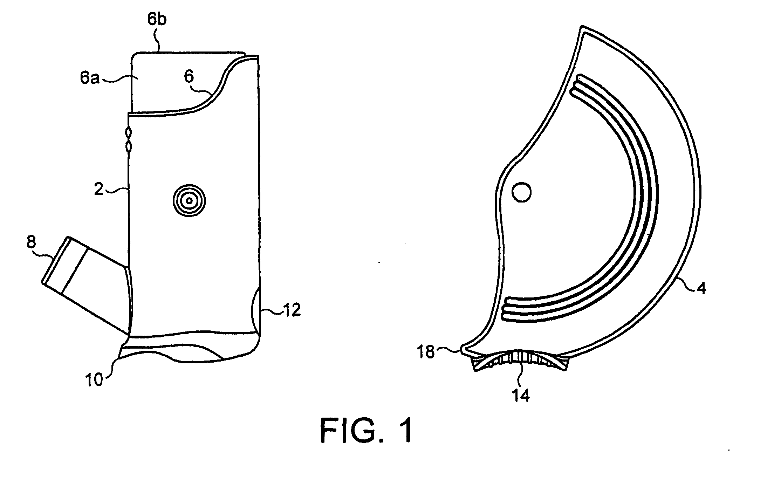

Referring now to the figures, Figure 1 illustrates a body 2 and a protective cover 4 for use in a medicament dispenser of the present invention. The body 2 has an opening 6 for insertion of the medicament container 6a and a valve (not shown). The discharge nozzle 8 is adjacent a protruding lip 10 for interacting with the cover 4. The body also has a grip 12.

The side view of the cover 4 illustrates the thumb grip 14 which is depressed and covered in ridges to avoid slippage.

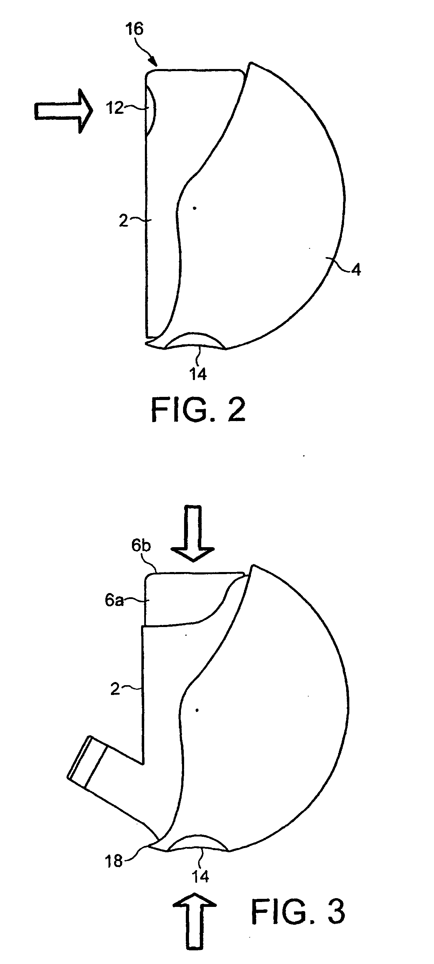

Figure 2 illustrates the dispenser 16 once assembled. The grips 12 and 14 are visible on the body and the cover respectively. In use, a patient holds the cover 4 in one hand and pushes on grip 12 on the body in the direction of the arrow. The body 2 thus rotates within the cover 4 until the dispensing position shown in figure 3 is achieved. Once the lip 10 reaches the edge 18 of cover 4, the body is prevented from further clockwise rotation. In order to dispense the medicament, a patient places his/her thumb under grip 14 and a forefinger on top of the medicament container 6a at the actuation point 6b and squeezes to push down the container 6a relative to its associated valve (not shown) as shown by the arrows in figure 3. The forces exerted between the grip 14 and the actuation point 6b dispenses a dose of medicament and also does not create any turning moments which would urge the body to turn anti-clockwise, that is towards the storage position. Therefore, the positioning of the body and the cover remains stable in the dispensing position.

Appropriate medicaments for dispensing may be selected from, for example, analgesics, e.g., codeine, dihydromorphine, ergotamine, fentanyl or morphine; anginal preparations, e.g., diltiazem; antiallergics, e.g., cromoglycate (eg s the sodium salt), ketotifen or nedocromil (eg as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (eg as the propionate ester), flunisolide, budesonide, rofleponide, mometasone eg as the furoate ester), ciclesonide, triamcinolone (eg as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, eg 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors eg (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g as free acid or potassium salt), diuretics, e.g., amiloride; anticholinergics, e.g., ipratropium (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an antiinflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It may be appreciated that any of the parts of the inhaler which contact the medicament suspension may be coated with materials such as fluoropolymer materials which reduce the tendency of medicament to adhere thereto. Any movable parts may also have coatings applied thereto which enhance their desired movement characteristics. Frictional coatings may therefore be applied to enhance frictional contact and lubricants used to reduce frictional contact as necessary.

It will be understood that the present crisclosure is for the purpose of illustration only and the invention extends to modifications, variations and improvements thereto within the scope of the following claims.

## Claims

1. A medicament dispenser comprising:-
(i) a medicament container (6a) having a dosing valve associated therewith such that medicament is dispensed in response to a force applied to an actuation point (6b) on said medicament container to depress said container relative to said valve;
(ii) a bodyl (2) for housing said container, said body having a discharge nozzle (8) for discharging the medicament therethrough; and
(iii) a protective cover (4) in which said body rotates between a storage position whereby said nozzle is covered and a dispensing position whereby said nozzle is exposed;
**characterised in that** said cover comprises a grip (14) positioned such that as the medicament is dispensed, opposing forces applied to said actuation point and to said grip do not generate a turning moment urging said body to return to said storage position.

2. A medicament dispenser according to claim 1 wherein the medicament container is an aerosol container.

3. A medicament dispenser according to claim 1 or claim 2 in the form of a nasal inhalator.

4. A medicament dispenser according to any one of claims 1 to 3 wherein the body further comprises a lip member (10) to restrain movement of the body with respect to the cover.

5. A medicament dispenser according to any one of claims 1 to 4 wherein the body further comprises a grip (12).

6. A medicament dispenser according to any one of the preceding claims wherein the grip on the cover and/or the body comprises ridges, ribs, depressions or mounds.

7. A medicament dispenser according to any one of the preceding claims wherein the grip on the cover and/or the body is made from plastics, and/or fabric, and/or rubber.

8. A medicament dispenser according to any one of the preceding claims wherein the cover is suspended on the body on an axis transverse to a longitudinal axis through the medicament container.

9. A medicament dispenser according to claim 8 wherein the axis is substantially central to the body and to the cover.

10. A medicament dispenser according to any one of the preceding claims wherein the cover and the body further comprise interacting members to retain the body in a dispensing or storage position.

11. A medicament dispenser according to any one of the preceding claims wherein said medicament container comprises a medicament formulation.

12. A medicament dispenser according to claim 11 wherein said medicament formulation comprises a medicament formulated as a suspension or solution in a liquified gas propellant.

13. A medicament dispenser according to either one of claims 11 or 12 wherein the medicament formulation comprises a medicament selected from the group consisting of albuterol, salmeterol, flucticasone propionate and beclomethasone dipropionate and salts or solvates thereof.

## Patentansprüche

1. Medikamentenspender, mit:
(i) einem Medikamentenbehälter (6a), der ein Dosierventil aufweist, das damit derart verbunden ist, dass ein Medikament aus Antwort auf eine Kraft abgegeben wird, die auf einen Betätigungspunkt (66) an dem Medikamentenbehälter angewandt wird, um den Behälter relativ zu dem Ventil herunterzudrücken;
(ii) einem Körper (2) zur Aufnahme des Behälters, wobei der Körper eine Auslassdüse (8) zum Ablassen des Medikaments **dadurch** aufweist; und
(iii) einer Schutzabdeckung (4), in welcher sich der Körper zwischen einer Lagerposition, wobei die Düse bedeckt ist, und einer Abgabeposition dreht, wobei die Düse offenliegt; **dadurch gekennzeichnet, dass** die Abdeckung einen Griff (14) umfasst, der derart positioniert ist, dass wenn das Medikament abgegeben wird, gegensätzliche Kräfte, die auf den Betätigungspunkt und den Griff angewandt werden, kein Drehmoment erzeugen, das den Körper dazu drängt in die Lagerposition zurückzukehren.

2. Medikamentenspender nach Anspruch 1, bei dem der Medikamentenbehälter ein Aerosolbehälter ist.

3. Medikamentenspender nach Anspruch 1 oder Anspruch 2, in der Form eines Naseninhalators.

4. Medikamentenspender nach einem der Ansprüche 1 bis 3, bei dem der Körper ferner ein Lippenelement (1a) umfasst, um eine Bewegung des Körpers bezüglich der Abdeckung einzuschränken.

5. Medikamentenspender nach einem der Ansprüche 1 bis 4, bei dem der Körper ferner einen Griff (12) umfasst.

6. Medikamentenspender nach einem der vorhergehenden Ansprüche, bei dem der Griff an der Abdeckung und/oder dem Körper Stege, Rippen, Vertiefungen oder Dämme umfasst.

7. Medikamentenspender nach einem der vorhergehenden Ansprüche, bei dem der Griff an der Abdeckung und/oder dem Körper aus Kunststoff und/oder Stoff und/oder Gummi hergestellt ist.

8. Medikamentenspender nach einem der vorhergehenden Ansprüche, bei dem die Abdeckung an dem Körper, an einer Achse transversal zu einer Längsachse durch den Medikamentenbehälter, aufgehängt ist.

9. Medikamentenspender nach Anspruch 8, bei dem die Achse im Wesentlichen zentral zu dem Körper und zu der Abdeckung ist.

10. Medikamentenspender nach einem der vorhergehenden Ansprüche, bei dem die Abdeckung und der Körper ferner sich gegenseitig beeinflussende Elemente umfassen, um den Körper in einer Abgabe- oder Lagerposition zu halten.

11. Medikamentenspender nach einem der vorhergehenden Ansprüche, bei dem der Medikamentenbehälter eine Medikamentenrezeptur umfasst.

12. Medikamentenspender nach Anspruch 11, bei dem die Medikamentenrezeptur ein Medikament mit einer Rezeptur umfasst, die als eine Suspension oder Lösung in einem Flüssiggas-Treibmittel angesetzt ist.

13. Medikamentenspender nach einem der Ansprüche 11 oder 12, bei dem die Medikamentenrezeptur ein Medikament umfasst, das aus der Gruppe ausgewählt wird, die aus Albuterol, Salmeterol, Fluticason-Propionat und Beclomethason-Dipropionat, und Salzen oder Solvaten davon, besteht.

## Revendications

1. Distributeur de médicament comprenant :
(i) un récipient à médicament (6a) comportant une soupape de dosage associée à ce dernier de telle manière que le médicament soit distribué en réponse à une force exercée sur un point d'activation (6b) sur ledit récipient à médicament pour abaisser par pression ledit récipient par rapport à ladite soupape ;
ii) un corps (2) pour loger ledit récipient, ledit corps comprenant une tubulure de sortie (8) pour distribuer le médicament à travers ce dernier ; et
iii) un couvercle de protection (4) dans lequel ledit corps tourne entre une position de rangement dans laquelle ladite tubulure est recouverte et une position de distribution dans laquelle ladite tubulure est exposée ;
**caractérisé en ce que** lequel ledit couvercle comporte un élément de préhension (14) positionné de manière à ce que lorsque le médicament est distribué, des forces opposées appliquées audit point d'activation et sur ledit élément de préhension ne génèrent pas de moment de rotation forçant ledit corps à revenir à ladite position de rangement.

2. Distributeur de médicament selon la revendication 1 dans lequel le récipient à médicament est un récipient à aérosol.

3. Distributeur de médicament selon la revendication 1 ou la revendication 2, sous la forme d'un inhalateur nasal.

4. Distributeur de médicament selon l'une quelconque des revendications 1 à 3 dans lequel le corps comprend en outre une lèvre (10) afin de limiter le déplacement du corps par rapport au couvercle.

5. Distributeur de médicament selon l'une quelconque des revendications 1 à 4 dans lequel le corps comprend en outre un élément de préhension (12).

6. Distributeur de médicament selon l'une quelconque des revendications précédentes dans lequel l'élément de préhension sur le couvercle et/ou le corps comporte des saillies, nervures, parties en creux ou en relief.

7. Distributeur de médicament selon l'une quelconque des revendications précédentes dans lequel l'élément de préhension sur le couvercle et/ou le corps est en matière plastique, et/ou en tissu, et/ou en caoutchouc.

8. Distributeur de médicament selon l'une quelconque des revendications précédentes dans lequel le couvercle est suspendu sur le corps sur un axe transversal à un axe longitudinal traversant le récipient à médicament.

9. Distributeur de médicament selon la revendication 8 dans lequel l'axe est sensiblement central par rapport au corps et au couvercle.

10. Distributeur de médicament selon l'une quelconque des revendications précédentes dans lequel le couvercle et le corps comprennent en outre des éléments coopérant mutuellement pour retenir le corps dans une position de distribution ou de rangement.

11. Distributeur de médicament selon l'une quelconque des revendications précédentes dans lequel ledit récipient à médicament contient une préparation médicamenteuse.

12. Distributeur de médicament selon la revendication 11 dans lequel ladite préparation médicamenteuse est constituée d'un médicament préparé sous forme d'une suspension ou d'une solution dans un gaz propulseur liquéfié.

13. Distributeur de médicament selon l'une quelconque des revendications 11 ou 12 dans lequel la préparation médicamenteuse est constituée d'un médicament choisi dans le groupe consistant en du salbutamol, du salmétérol, du propionate de fluticasone et du dipropionate de béclométhasone, ainsi que les sels et solvats de ces derniers.
